# EUROPÄISCHE PATENTSCHRIFT

(11) **EP 3 890 870 B1**
(45) Veröffentlichungstag und Bekanntmachung des Hinweises auf die Patenterteilung: **14.10.2026**
(21) Anmeldenummer: 19816306.5
(22) Anmeldetag: 05.12.2019
(51) Int. Cl.: B01D 61/14, A23C 9/142, A61M 1/34, B01D 61/16, B01D 61/18, C07K 1/34, C07K 1/36

(54) **VORRICHTUNG UND VERFAHREN ZUR MEHRFACHEN ÄNDERUNG DER ZUSAMMENSETZUNG EINES FLUIDS**
DEVICE AND METHOD FOR REPEATEDLY MODIFYING THE COMPOSITION OF A FLUID
PROCEDE ET DISPOSITIF POUR MODIFIER PLUSIEURS FOIS LA COMPOSITION D'UN FLUIDE

(30) Priorität: 07.12.2018 DE 102018009597
(43) Veröffentlichungstag der Anmeldung: 13.10.2021
(73) Patentinhaber: Sartorius Stedim Biotech GmbH, 37079 Göttingen (DE)
(72) Erfinder: LEUTHOLD, Martin, 37079 Göttingen (DE); HELLING, Alexander, 37136 Ebergötzen (DE); GRUMMERT, Ulrich, 37242 Bad Sooden-Allendorf (DE)
(74) Vertreter: Novagraaf International SA
(86) Internationale Anmeldenummer: PCT/EP2019/083797
(87) Internationale Veröffentlichungsnummer: WO 2020/115196

(56) Entgegenhaltungen:
- EP-A1- 3 088 006
- WO-A1-2009/129226
- WO-A1-89/02707
- WO-A2-2015/175679
- DE-A1- 102016 004 115
- DE-T3- 69 001 344
- US-A1- 2007 246 406
- US-A1- 2008 237 110

## Beschreibung

Die vorliegende Erfindung betrifft eine Vorrichtung und ein Verfahren zur mehrfachen Änderung der Zusammensetzung eines Fluids.

In einer Vielzahl von Prozessen ist es erforderlich, die Zusammensetzung eines Fluids mehrfach zu ändern. Bei bestehenden Verfahren kann hierzu jedoch eine Volumenänderung des Fluids erforderlich sein, was die Verfahrensführung erschweren kann. Es ist allerdings wünschenswert, das Volumen des Fluids im Verfahren beliebig ändern zu können. Zudem ist mit herkömmlichen Verfahren die simultane Abtrennung von Verunreinigungen mit einem erhöhten Aufwand verbunden.

DE 10 2016 004 115 A1 beschreibt eine Crossflow-Filtrationseinheit zur kontinuierlichen Diafiltration.

WO 2018/039163 A1 beschreibt ein Verfahren zur Aufreinigung eines heterologen Proteins aus Eiklar.

EP 3 116 552 A1 beschreibt eine Vorrichtung und ein Verfahren zur kontinuierlichen Virusinaktivierung.

EP 3 288 596 B1 beschreibt ein Verfahren zur kontinuierlichen Virusinaktivierung in einem Mikroreaktor.

Somit liegt der vorliegenden Erfindung die Aufgabe zugrunde, eine Vorrichtung sowie ein Verfahren, welche ohne Einschränkungen hinsichtlich der Volumenänderung des Fluids eine schonende mehrfache Änderung der Zusammensetzung eines Fluids bei gleichzeitiger Entfernung von Verunreinigungen ermöglichen sollen, bereitzustellen.

Die vorstehende Aufgabe wird durch die in den Ansprüchen gekennzeichneten Ausführungsformen gelöst.

In einem ersten Aspekt betrifft die vorliegende Erfindung eine Vorrichtung gemäß Anspruch 1. Des weiteren betrifft die Erfindung ein Verfahren gemäß Anspruch 4 und Verwendungen der Vorrichtung gemäß Ansprüchen 12 und 13.

Die erfindungsgemäße Vorrichtung ermöglicht es, auf schonende Weise die Zusammensetzung eines Fluids mehrfach zu ändern und gleichzeitig Verunreinigungen in dem Fluid abzutrennen. Insbesondere eignen sich die erfindungsgemäße Vorrichtung und das erfindungsgemäße Verfahren dazu, die Zusammensetzung eines Fluids für einen kontrollierbaren Zeitraum zu ändern. Zudem kann gleichzeitig mit den Änderungen der Zusammensetzung auch das Volumen des Fluids eingestellt werden. Die erfindungsgemäße Vorrichtung und das erfindungsgemäße Verfahren sind darüber hinaus für eine kontinuierliche Betriebsweise geeignet.

Unter einem Fluid wird erfindungsgemäß ein flüssiges oder gasförmiges Gemisch oder eine flüssige oder gasförmige Verbindung verstanden. Beispielsweise stellt eine Pufferlösung ein Fluid dar. Auch ein Kulturmedium oder dessen flüssige bzw. gasförmige Bestandteile können ein Fluid sein.

Gemäß der vorliegenden Erfindung ist zumindest eines von dem ersten und dem zweiten Modul die vorstehend definierte Filtereinheit. Darüber hinaus unterliegen das erste und zweite Modul keiner besonderen Einschränkung. Das heißt, es kann jedwede zur Änderung der Zusammensetzung eines Fluids geeignete Vorrichtung verwendet werden.

Gemäß einer bevorzugten Ausführungsform der vorliegenden Erfindung ist das erste oder zweite Modul ein statischer Mischer. Dadurch entfällt für das Modul, welches ein statischer Mischer ist, die Notwendigkeit, einen Permeatstrom abzuführen, wodurch das Verfahren einfacher durchgeführt werden kann und besonders wirtschaftlich ist. Vorstehende bevorzugte Ausführungsform umfasst den Fall, worin das erste Modul ein statischer Mischer ist und das zweite Modul die Filtereinheit ist, und den Fall, worin das erste Modul die Filtereinheit ist und das zweite Modul ein statischer Mischer ist. Der Fall, worin das erste Modul ein statischer Mischer ist und das zweite Modul die Filtereinheit ist, ist bevorzugt.

Gemäß einer weiteren bevorzugten Ausführungsform der vorliegenden Erfindung ist das erste oder zweite Modul die vorstehend beschriebene Filtereinheit mit erstem und zweitem Filtermedium und das zweite oder erste Modul eine weitere Filtereinheit, aufweisend mindestens ein Filtermedium. Vorzugsweise weist die weitere Filtereinheit ein einziges Filtermedium auf. Die weitere Filtereinheit kann beispielsweise ein Sterilfilter sein. Vorzugsweise weist die weitere Filtereinheit lediglich einen Einlass und lediglich einen Auslass auf (Dead-End-Filter). Es ist bevorzugt, dass das erste Modul die vorstehend beschriebene Filtereinheit mit erstem und zweitem Filtermedium ist und das zweite Modul die weitere Filtereinheit mit mindestens einem Filtermedium ist. Mit einer erfindungsgemäßen Vorrichtung dieser Ausführungsform kann beispielsweise mit dem ersten Modul eine Fällungsreaktion ausgelöst werden, wobei die Fällungsreaktion im Verweilmodul ablaufen kann und die weitere Filtereinheit das Fällungsprodukt abtrennen kann.

Gemäß einer weiteren bevorzugten Ausführungsform der vorliegenden Erfindung ist das erste oder zweite Modul die vorstehend beschriebene Filtereinheit mit erstem und zweitem Filtermedium und das zweite oder erste Modul eine weitere Filtereinheit, aufweisend mindestens einen Versorgungskanal, mindestens ein Filtermedium und mindestens einen Retentatkanal, derart angeordnet, dass das Filtermedium den Versorgungskanal und den Retentatkanal voneinander abgrenzt, wobei der Versorgungskanal fluidleitend mit mindestens einem Einlass für ein Versorgungsmedium verbunden ist und der Retentatkanal fluidleitend mit mindestens einem Einlass für das Fluid und mit mindestens einem Auslass für das Fluid verbunden ist. Die Filtereinheit mit Filtermedium ist wie die vorstehend beschriebene Filtereinheit mit erstem und zweitem Filtermedium aufgebaut, mit der Maßgabe, dass das zweite Filtermedium sowie der Permeatkanal und dessen Auslass fehlen. Für diese weitere Filtereinheit gelten die Ausführungen in Bezug auf die Filtereinheit mit erstem und zweitem Filtermedium entsprechend. Die weitere Filtereinheit kann insbesondere dann verwendet werden, wenn das Volumen des Fluids durch das erste oder zweite Modul nicht kontrolliert werden soll. Gemäß dieser Ausführungsform kann mit der Filtereinheit mit Filtermedium beispielsweise eine Verdünnung durchgeführt werden.

Gemäß einer weiteren bevorzugten Ausführungsform der vorliegenden Erfindung ist das erste oder zweite Modul die vorstehend beschriebene Filtereinheit mit erstem und zweitem Filtermedium und das zweite oder erste Modul eine weitere Filtereinheit, aufweisend mindestens einen Retentatkanal, mindestens ein Filtermedium und mindestens einen Permeatkanal, derart angeordnet, dass das Filtermedium den Retentatkanal und den Permeatkanal voneinander abgrenzt, wobei der Retentatkanal fluidleitend mit mindestens einem Einlass für das Fluid und mit mindestens einem Auslass für das Fluid verbunden ist und der Permeatkanal fluidleitend mit mindestens einem Auslass für das Permeat verbunden ist. Die Filtereinheit mit Filtermedium ist wie die vorstehend beschriebene Filtereinheit mit erstem und zweitem Filtermedium aufgebaut, mit der Maßgabe, dass das erste Filtermedium sowie der Versorgungskanal und dessen Einlass fehlen. Für diese weitere Filtereinheit gelten die Ausführungen in Bezug auf die Filtereinheit mit erstem und zweitem Filtermedium entsprechend. Die weitere Filtereinheit kann insbesondere dann verwendet werden, wenn das Volumen des Fluids durch das erste oder zweite Modul nicht kontrolliert werden soll. Zudem kann gemäß dieser Ausführungsform mit der weiteren Filtereinheit mit Filtermedium beispielsweise ein Fällungsprodukt abgetrennt werden.

Gemäß einer weiteren bevorzugten Ausführungsform der vorliegenden Erfindung ist das erste oder zweite Modul die vorstehend beschriebene Filtereinheit mit erstem und zweitem Filtermedium und das zweite oder erste Modul ein Membranadsorber. Vorzugsweise ist das erste Modul ein Membranadsorber und das zweite Modul die Filtereinheit mit erstem und zweitem Filtermedium. In diesem Fall kann der Membranadsorber zunächst mit einem Produkt (z. B. monoklonaler Antikörper, Protein) beladen werden. Hierzu wird dem Membranadsorber zunächst ein Beladungsmedium, welches das Produkt enthält, zugeführt. Dadurch wird das im Beladungsmedium enthaltene Produkt vom Membranadsorber adsorbiert. Das Beladungsmedium kann die erfindungsgemäße Vorrichtung über das Verweilmodul und das zweite Modul (Filtereinheit) verlassen. Vorzugsweise ist am Auslass des Membranadsorbers ein Ventil angebracht, sodass das Beladungsmedium darüber abgeführt werden kann, ohne das Verweilmodul und das zweite Modul passieren zu müssen. Nachdem das Beladungsmedium abgeführt wurde, kann dem Membranadsorber das Fluid als Eluens zugeführt werden, sodass der Membranadsorber das daran adsorbierte Produkt an das Fluid abgibt. In diesem Fall weist das Fluid, welches dem Membranadsorber zugeführt wird, vorzugsweise einen pH-Wert von weniger als 7, besonders bevorzugt von 2 bis 6, insbesondere bevorzugt von 3 bis 5, auf. Dadurch können im Fluid gegebenenfalls enthaltene Viren inaktiviert werden.

Gemäß einer weiteren bevorzugten Ausführungsform der vorliegenden Erfindung kann anstelle eines Membranadsorbers ein Chromatographiemodul, welches ein Chromatographiemedium enthält, verwendet werden. Das Chromatographiemedium kann beispielsweise ein Gel oder ein Monolith sein. Vorstehende Ausführungen in Bezug auf die Ausführungsform mit Membranadsorber gelten für die Ausführungsform mit Chromatographiemodul entsprechend.

Gemäß einer besonders bevorzugten Ausführungsform der vorliegenden Erfindung ist das erste Modul eine erste Filtereinheit und das zweite Modul eine zweite Filtereinheit. Dadurch kann eine besonders schonende mehrfache Zusammensetzungsänderung erzielt werden. Zudem kann sowohl durch das erste Modul als auch durch das zweite Modul das Volumen des Fluids eingestellt werden. Gleichermaßen ermöglicht es diese bevorzugte Ausführungsform, Verunreinigungen besonders wirksam zu entfernen.

Die erfindungsgemäße Vorrichtung kann mit Sensoren versehen sein. Zu geeigneten Sensoren zählen Sensoren zur Messung des pH-Werts (pH-Sensoren), der Leitfähigkeit, des Drucks und des Flusses sowie Spektroskopie-Sensoren (UV-, UV/VIS-, IR-, NIR- sowie Raman-Spektroskopie).

Die Sensoren dienen zur Überwachung und Steuerung des erfindungsgemäßen Verfahrens. Vorzugsweise sind am Auslass (8, 17) des Retentatkanals Sensoren angebracht, insbesondere bevorzugt pH-Sensoren. Damit können die Zusammensetzung oder andere Eigenschaften des Fluids bei Verlassen der Filtereinheit des ersten bzw. zweiten Moduls gemessen werden.

Die erfindungsgemäße Vorrichtung kann Ventile und/oder Pumpen aufweisen. Mit den Ventilen und/oder Pumpen können die Stoffströme des Fluids, des Versorgungsmediums und des Permeats gesteuert werden. Geeignete Ventile sind beispielsweise Quetschventile und Nadelventile. Vorzugsweise ist am Auslass des Retentatkanals ein Ventil oder eine Pumpe angebracht. Durch das Ventil bzw. die Pumpe kann der Filtrationsdruck innerhalb des Retentatkanals und somit der Volumenstrom durch das zweite Filtermedium eingestellt werden. Davon unabhängig ist bevorzugt, dass der Auslass des Permeatkanals ein Ventil aufweist. Durch das Ventil an seinem Auslass kann der Permeatkanal geschlossen werden. Dies kann hilfreich sein, wenn in der Filtrationseinheit lediglich eine Vereinigung des Versorgungsmediums und des Fluids gewünscht ist, ohne eine Filtration durch das zweite Filtermedium durchzuführen. Die Filtereinheit(en) mit erstem und zweitem Filtermedium können im Retentat-, Versorgungs- und Permeatkanal, vorzugsweise im Retentatkanal, mit Einbauten ausgestattet sein. Derartige Einbauten sind dazu geeignet, den Retentat-, Versorgungs- und Permeatkanal offen zu halten und/oder eine optimale Durchmischung im jeweiligen Kanal zu gewährleisten. Die Einbauten können beispielsweise aus einem Textilmaterial (z. B. Gewebe, Gewirke und/oder Vlies) bestehen.

Das Verweilmodul unterliegt erfindungsgemäß keiner besonderen Einschränkung, solange es dazu geeignet ist, ein Fluid aufzunehmen und wieder abzugeben. Durch Größe und Gestalt des Verweilmoduls kann die Dauer zwischen der Zusammensetzungsänderung im ersten Modul und Zusammensetzungsänderung im zweiten Modul eingestellt werden. Das Verweilmodul kann beispielsweise ein Behälter mit Einlass und Auslass sein. Ein konkretes Beispiel für das Verweilmodul ist ein Rührkessel mit einem Einlass zum Zuführen des Fluids und einem Auslass zum Abführen des Fluids. Gemäß einer bevorzugten Ausführungsform der vorliegenden Erfindung ist das Verweilmodul so eingerichtet, dass die Verweilzeit des Fluids in dem Verweilmodul 1 Minute bis 24 Stunden, vorzugsweise 5 Minuten bis 2 Stunden, bevorzugter 10 Minuten bis 1 Stunde, beträgt.

Das Verweilmodul definiert einen Fluidpfad. Ein Fluidpfad ist ein Volumen, welches von dem Fluid durchströmt werden kann, solange es sich im Verweilmodul befindet. Ein Verweilmodul, welches einen Fluidpfad definiert, kann beispielsweise ein Rohr oder ein Schlauch sein. In diesem Fall bildet, einem Rohrreaktor vergleichbar, ein Ende des Rohrs oder Schlauches den Einlass, das andere Ende den Auslass des Verweilmoduls.

Der Fluidpfad kann beispielsweise einen oder mehrere mäanderförmige Kanäle aufweisen und/oder einen oder mehrere spiralförmige Kanäle. Die einen oder mehreren Kanäle können beispielsweise durch Drucken, Fräsen und/oder Spritzguss in eine Kunststoffoberfläche eingebracht sein oder durch einen entsprechend geformten Schlauch bzw. ein entsprechend geformtes Rohr gebildet sein.

Gemäß einer bevorzugten Ausführungsform der vorliegenden Erfindung umfasst das Verweilmodul ein zusammenhängendes Volumen, welches durch mindestens ein Unterteilungselement unterteilt ist. Ein derart unterteiltes und zusammenhängendes Volumen definiert einen Fluidpfad, der von dem Fluid durchströmt werden kann. Das mindestens eine Unterteilungselement kann beispielsweise aus einer Mehrzahl von Umlenkplatten und/oder Umlenkfolien bestehen.

Bei der Verwendung von Umlenkfolien sind vorzugsweise zwischen den Umlenkfolien Abstandshalter eingebracht. Dadurch kann die mechanische Stabilität bzw. Ortsfestigkeit der Umlenkfolien erhöht werden. Als Abstandshalter wird vorzugsweise ein Textilmaterial (z. B. Gewebe, Gewirke und/oder Vlies) verwendet. Durch ein Textilmaterial können eine gründliche Durchmischung des Fluids gewährleistet und das Auftreten von Bereichen mit laminarer Strömung reduziert werden. Ein Abstandshalter/Textilmaterial kann auch dann in den vorzugsweise von dem Verweilmodul definierten Fluidpfad eingebracht sein, wenn keine Umlenkplatten oder -folien verbaut sind.

Gemäß einer weiteren bevorzugten Ausführungsform der vorliegenden Erfindung umfasst das Verweilmodul mehrere parallele Strömungskanäle. Jeder der parallelen Strömungskanäle ist mit einem gemeinsamen Einlass und mit einem gemeinsamen Auslass für das Fluid verbindbar. Dadurch können ein oder mehrere der parallelen Strömungskanäle mit dem gemeinsamen Einlass und Auslass verbunden werden (Fluid durchströmt den verbundenen Strömungskanal) oder von dem gemeinsamen Einlass und Auslass abgesperrt werden (durch den abgesperrten Strömungskanal fließt kein Fluid). Somit ist es möglich, die Anzahl der parallelen Strömungskanäle, durch die das Fluid fließt, zu ändern, wodurch die Verweilzeit des Fluids im Verweilmodul reguliert werden kann. Die Verbindbarkeit der parallelen Strömungskanäle mit dem gemeinsamen Einlass und mit dem gemeinsamen Auslass für das Fluid kann beispielsweise mithilfe von Ventilen gewährleistet werden.

An das zweite Modul kann sich gegebenenfalls ein weiteres Verweilmodul, welches fluidleitend mit einem Auslass des zweiten Moduls verbunden ist, anschließen. Mit dem Auslass des weiteren Verweilmoduls kann ein drittes Modul, welches wie das erste bzw. zweite Modul aufgebaut sein kann, fluidleitend verbunden sein. Durch eine derartige Anordnung kann eine Kaskade von Modulen zur Zusammensetzungsänderung und Verweilmodulen aufgebaut werden, wodurch die Zusammensetzung des Fluids mehr als zwei Mal geändert werden kann.

Die erfindungsgemäße Vorrichtung kann mehr als ein erstes Modul, mehr als ein zweites Modul und/oder mehr als ein Verweilmodul aufweisen. Das heißt, die erfindungsgemäße Vorrichtung kann zwei oder mehr erste Module, zwei oder mehr zweite Module und/oder zwei oder mehr Verweilmodule aufweisen. Durch eine derartige Parallelisierung kann der Durchsatz des ersten Moduls, des zweiten Moduls und/oder des Verweilmoduls angepasst werden.

In der erfindungsgemäßen Vorrichtung ist das erste Modul mit dem Einlass des Verweilmoduls fluidleitend verbunden. Zudem ist der Auslass des Verweilmoduls fluidleitend mit dem zweiten Modul verbunden. Dabei kann das erste Modul direkt (d. h. ohne Zwischenverbindung) mit dem Einlass des Verweilmoduls verbunden sein oder über eine Zwischenverbindung, beispielsweise über eine Rohrleitung oder einen Schlauch. Das gleiche gilt für die Verbindung zwischen dem Auslass des Verweilmoduls und dem zweiten Modul. Bei einer direkten Verbindung kann eine kompakte Bauweise der erfindungsgemäßen Vorrichtung realisiert werden. Bei Verwendung einer Zwischenverbindung kann die erfindungsgemäße Vorrichtung in einfacher Weise aus erstem und zweiten Modul sowie Verweilmodul zusammengebaut werden und bei Bedarf mit Modulen verschiedener Bauart kombiniert werden.

Sofern das erste Modul eine wie vorstehend beschriebene Filtereinheit mit erstem und zweitem Filtermedium ist, dann ist der Auslass des Retentatkanals des ersten Moduls fluidleitend mit dem Einlass des Verweilmoduls verbunden. Sofern das zweite Modul eine wie vorstehende beschriebene Filtereinheit mit erstem und zweitem Filtermedium ist, dann ist der Auslass des Verweilmoduls fluidleitend mit dem Einlass des Retentatkanals des zweiten Moduls verbunden.

Gemäß der vorliegenden Erfindung umfasst die Vorrichtung als erstes und/oder zweites Modul die vorstehend beschriebene Filtereinheit mit erstem und zweitem Filtermedium. Hierin gelten Ausführungen in Bezug auf derartige Filtereinheiten sowohl für den Fall, dass das erste und das zweite Modul derartige Filtereinheiten sind, als auch für den Fall, dass nur eines von dem ersten und zweiten Modul eine derartige Filtereinheit ist.

Gemäß einer bevorzugten Ausführungsform der vorliegenden Erfindung ist die Filtereinheit ein Flachfilter-, Wickel- oder Hohlfasermodul, wobei ein Flachfiltermodul bevorzugt ist. Sofern die Filtereinheit als Flachfiltermodul ausgestaltet ist, haben das erste Filtermedium und das zweite Filtermedium eine flächige Form. Der Begriff "flächig" zeigt an, dass das jeweilige Filtermedium (Filtermaterial) im Wesentlichen in einer einzigen Ebene liegt. Vorzugsweise liegen alle Filtermedien im Wesentlichen in Ebenen, die weitgehend parallel zueinander sind. Die erfindungsgemäß geeigneten Filtermedien unterliegen keiner besonderen Einschränkung und können beispielsweise Keramikmembranen, Vliese und Polymermembranen sein.

Die ersten und zweiten Filtermedien sind zur Filtration des Versorgungsmediums und des Fluids geeignet. Das erste und zweite Filtermedium sind zumindest teilweise für das Versorgungsmedium und das Fluid durchlässig.

Gemäß einer bevorzugten Ausführungsform ist zumindest das zweite Filtermedium für ein oder mehrere gegebenenfalls in dem Fluid enthaltene Produkte nicht durchlässig. Dadurch wird verhindert, dass gegebenenfalls im Fluid enthaltene Produkte von dem Retentatkanal in den Permeatkanal geraten. Vorzugsweise ist auch das erste Filtermedium für die im Fluid gegebenenfalls enthaltenen Produkte nicht durchlässig. Wie nachstehend genauer erläutert, können die Produkte beispielsweise ein Antikörper sein.

Vorzugsweise weisen das erste Filtermedium und das zweite Filtermedium unabhängig voneinander jeweils eine Porengröße von 0,5 nm bis 1 mm, besonders bevorzugt 1 nm bis 10 µm, insbesondere bevorzugt 2 nm bis 100 nm, auf. "Unabhängig voneinander" bedeutet in diesem Zusammenhang, dass erstes und zweites Filtermedium nicht die gleiche Porengröße und/oder Materialeigenschaften aufweisen müssen.

Zur Bestimmung der Porengröße wird erfindungsgemäß bei Porengrößen von mindestens 0,1 µm, d. h. für Mikrofiltrationsmembranen mit einer mittleren Porengröße von 0,1 bis 10 µm, die Kapillarfluss-Porometrie verwendet. Hierbei handelt es sich um eine Gas/Flüssigkeits-Porosimetrie, bei der die differentiellen Gasdrücke und Flussraten durch eine Membranprobe zuerst im feuchten und anschließend im trockenen Zustand gemessen werden. Die Membranprobe wird vor der Messung mit einer benetzenden Flüssigkeit so in Kontakt gebracht, dass sämtliche vorhandenen Poren mit dieser Flüssigkeit gefüllt sind. Nach dem Füllen der Poren und dem Einbringen der Probe ist die Messzelle zu verschließen und die Messung zu starten. Der Gasdruck wird nach dem Start der Messung automatisch und schrittweise erhöht und die dem anliegenden Druck entsprechenden Porendurchmesser werden durch den Gasdruck entleert. Dies erfolgt solange, bis der relevante Porenbereich erfasst wurde, d. h. bis auch die kleinsten im Messbereich vorhandenen Poren von der Flüssigkeit befreit sind. Danach wird der Druck wieder abgesenkt und die Messung an der nun trockenen Probe automatisch wiederholt. Aus der Differenz beider Druck-Flussrate-Kurven wird die Porengrö-ßenverteilung über die Young-Laplace-Gleichung berechnet (s. auch A. Shrestha, "Characterization of porous membranes via porometry", 2012, Mechanical Engineering Graduate Theses & Dissertations, Paper 38, University of Colorado at Boulder).

Zur Bestimmung von Porengrößen von mehr als 10 µm bis 1 mm kann das in Journal of Membrane Science 372 (2011), Seiten 66 bis 74, beschriebene, auf Bildanalyse (image analysis) beruhende Verfahren verwendet werden.

Bei Porengrößen von weniger als 0,1 µm wird erfindungsgemäß die Flüssig-Flüssig-Verdrängungsmethode angewendet. Diese weist Ähnlichkeiten zur Kapillarfluss-Porometrie auf. Allerdings werden hier nicht die Gasflussraten, sondern die Flussraten der verdrängenden Flüssigkeit in Abhängigkeit der differentiellen Druckerhöhung gemessen (s. auch R. Dävila, "Characterization of ultra and nanofiltration commercial filters by liquid-liquid displacement porosimetry", 2013).

Gemäß einer bevorzugten Ausführungsform der Erfindung ist das erste Filtermedium eine erste Filtrationsmembran. Das zweite Filtermedium ist vorzugsweise eine zweite Filtrationsmembran. Besonders bevorzugt ist das erste Filtermedium eine erste Filtrationsmembran und das zweite Filtermedium ist eine zweite Filtrationsmembran. Die Filtrationsmembranen können beispielsweise aus Polyvinylidenfluorid, Cellulose und deren Derivaten, Polyethersulfon (PES) oder Polysulfon bestehen, wobei vernetztes Cellulosehydrat besonders bevorzugt ist.

Die erfindungsgemäße Vorrichtung eignet sich für alle Verwendungen, in denen die Zusammensetzung eines Fluids mehrmals geändert werden muss. Insbesondere ist die erfindungsgemäße Vorrichtung zur Virusinaktivierung geeignet.

Die nachstehenden Ausführungen in Bezug auf das erfindungsgemäße Verfahren gelten für die erfindungsgemäße Vorrichtung entsprechend.

In einem weiteren Aspekt betrifft die vorliegende Erfindung ein Verfahren zur mehrfachen Änderung der Zusammensetzung eines Fluids, umfassend (a) Bereitstellen der vorstehend beschriebenen erfindungsgemäßen Vorrichtung; (b) Zuführen des Fluids in das erste Modul; und (c) Abführen des Fluids aus dem zweiten Modul, wobei Schritt (b) und/oder Schritt (c) die folgenden Schritte umfasst/umfassen: (i) Zuführen des Versorgungsmediums in den Einlass für das Versorgungsmedium; (ii) Zuführen des Fluids in den Einlass für das Fluid; (iii) Abführen des Fluids aus dem Auslass für das Fluid; und (iv) Abführen des Permeats aus dem Auslass für das Permeat. Dadurch, dass in der erfindungsgemäßen Vorrichtung das erste Modul mit dem Einlass des Verweilmoduls fluidleitend verbunden ist und der Auslass des Verweilmoduls fluidleitend mit dem zweiten Modul verbunden ist, fließt das Fluid im erfindungsgemäßen Verfahren zunächst durch das erste Modul, dann durch das Verweilmodul und schließlich durch das zweite Modul.

Die vorstehenden Ausführungen in Bezug auf die erfindungsgemäße Vorrichtung gelten für das erfindungsgemäße Verfahren entsprechend.

Das erfindungsgemäße Verfahren eignet sich für beliebige Anwendungen, worin die mehrmalige Änderung der Zusammensetzung eines Fluids erforderlich ist. Es ist beispielsweise möglich, im ersten Modul das Fluid mit einem Reaktanden in Kontakt zu bringen, um eine chemische Reaktion auszulösen, im Verweilmodul die chemische Reaktion ablaufen zu lassen und im zweiten Modul die Reaktion, beispielsweise durch Zugabe eines weiteren Reaktanden, abzubrechen (zu quenchen).

Durch Schritte (i) bis (iv) des erfindungsgemäßen Verfahrens kann zugleich eine schonende Änderung der Zusammensetzung des Fluids als auch eine Abtrennung von Verunreinigungen von dem Fluid erzielt werden. Das zweite Filtermedium kann so gewählt werden, dass die gegebenenfalls im Fluid enthaltenen Verunreinigungen das zweite Filtermedium passieren, um als Bestandteile des Permeats abgeführt zu werden, wobei erwünschte Produkte (z. B. monoklonale Antikörper) im Fluid verbleiben, weil sie das zweite Filtermaterial nicht passieren können. Alternativ kann das zweite Filtermedium so gewählt werden, dass es von unerwünschten Verunreinigungen nicht passiert werden kann. In diesem Fall kann das zweite Filtermedium vom Produkt passiert werden. In diesem Fall verbleiben die unerwünschten Verunreinigungen im Fluid. Das heißt, auch das Permeat kann das am Ende des Verfahrens erwünschte Produkt sein bzw. enthalten.

Besonders eignet sich das erfindungsgemäße Verfahren dazu, durch Änderung der Zusammensetzung im ersten Modul eine Fällungsreaktion auszulösen, die Fällungsreaktion im Verweilmodul ablaufen zu lassen und im zweiten Modul das Fällungsprodukt abzutrennen, beispielsweise durch Filtration.

Darüber hinaus eignet sich das erfindungsgemäße Verfahren dazu, im ersten Modul einen Katalysator zum Fluid zu geben, im Verweilmodul eine durch den Katalysator katalysierte Reaktion ablaufen zu lassen und im zweiten Modul den Katalysator vom Fluid abzutrennen. Der Katalysator unterliegt keiner besonderen Einschränkung. Vorzugsweise ist der Katalysator ein Enzym (Biokatalysator). Konkret eignet sich das erfindungsgemäße Verfahren für die Erzeugung von laktosefreier Milch. Hierzu wird im ersten Modul über den Versorgungskanal Laktase zum Fluid, welches in diesem Fall laktosehaltige Milch ist, zugegeben. Im Verweilmodul setzt die Laktase die im Fluid enthaltene Laktose um. Im zweiten Modul wird die Laktase abgetrennt. Bei Verwendung einer Filtereinheit mit erstem und zweitem Filtermedium kann die laktosefreie Milch als Permeat abgetrennt werden, wobei die Laktase im Rententat verbleibt.

Gemäß einer bevorzugten Ausführungsform der vorliegenden Erfindung enthält das Fluid, welches in das erste Modul eingeleitet wird, ein oder mehrere Produkte sowie gegebenenfalls eine oder mehrere Verunreinigungen.

Das oder die Produkte unterliegen keiner besonderen Einschränkung. Vorzugsweise stammt das Produkt aus einem biotechnologischen Verfahren, vorzugsweise wird es von Zellen hergestellt. Zu "Zellen" zählen erfindungsgemäß solche, die Menschen, Tiere, Pflanzen, Pilze, Algen und Bakterien als Ursprung haben. Ebenso ist erfindungsgemäß bevorzugt, dass das Produkt ein Protein (z. B. ein Antikörper), Virus und/oder ein Vakzin ist.

Das Protein unterliegt keiner besonderen Einschränkung und kann beispielsweise ein Enzym oder ein Antikörper sein, wobei das Protein vorzugsweise ein oder mehrere Antikörper sind. Konkrete Beispiele für den Antikörper sind Immunglobulin A, D, E, G, Y und deren Gemische. Der Antikörper kann beispielsweise rekombinant, polyklonal oder monoklonal sein.

Des Weiteren kann das Produkt ein Virus oder ein Vakzin sein. Der Virus kann beispielsweise ein modifizierter Virus sein, z. B. ein Virus zur Gentherapie. Viren zur Gentherapie befallen Zellen des absichtlich mit dem Virus infizierten Patienten und bringen ihre RNS bzw. DNS in die Zelle ein. Auch Vakzine können Viren bzw. Virus-Bruchstücke umfassen. Sowohl das Produkt als auch die nachstehend genauer beschriebenen Verunreinigungen können Viren umfassen, wobei in diesem Fall die Produktviren von den Verunreinigungsviren verschieden sind. Grundsätzlich ist es möglich, die Produktviren von den Verunreinigungsviren zu trennen, beispielsweise durch Filtration (falls Produkt- und Verunreinigungsviren unterschiedliche Größen aufweisen). Alternativ oder ergänzend dazu können grundsätzlich durch das erfindungsgemäße Verfahren selektiv die Verunreinigungsviren inaktiviert werden. Beispielsweise ist es möglich, dass die Verunreinigungsviren bei einem pH-Wert inaktiviert werden, bei dem die Produktviren noch nicht inaktiviert werden. So können nur die Verunreinigungsviren inaktiviert werden, ohne die Produktviren zu inaktivieren.

Die gegebenenfalls enthaltenen Verunreinigungen sind unerwünscht. Sie können gegebenenfalls mithilfe von Filtration vom Produkt getrennt werden, beispielsweise durch die Filtereinheit mit erstem und zweitem Filtermedium. Alternativ bzw. ergänzend dazu können die Verunreinigungen gegebenenfalls durch das erfindungsgemäße Verfahren inaktiviert, zerstört oder in anderer Weise unschädlich gemacht werden. Konkret können die Verunreinigungen Viren sein (welche von den gegebenenfalls im Fluid enthaltenen erwünschten Produktviren verschieden sind). Beispiele für Verunreinigungen umfassen neben Viren Salze, DNS, HCP (*host cell protein*, Wirtszellenprotein), Zucker, sowie Aggregate (z. B. Proteinaggregate).

Für gewisse Produkte (beispielsweise pharmazeutische Erzeugnisse wie aus Zellen hergestellte monoklonale Antikörper) kann es aus Gründen der Hygiene erforderlich sein, das Fluid, worin unerwünschte Viren als Verunreinigungen enthalten sein können, einer Virusinaktivierung zu unterziehen. Diese Virusinaktivierung kann durch eine zeitlich beschränkte Änderung des pH-Werts des antikörperhaltigen Fluids erzielt werden, wie nachstehend beschrieben.

In einer bevorzugten Ausführungsform des erfindungsgemäßen Verfahrens wird im ersten Modul der pH-Wert des Fluids abgesenkt bzw. erhöht, wobei im zweiten Modul der pH-Wert des Fluids erhöht bzw. abgesenkt wird. Derartige Änderungen des pH-Wertes können durch Zugabe einer Säure bzw. einer Base im ersten und zweiten Modul erzielt werden. Dabei kann der anfängliche pH-Wert des Fluids, welches dem ersten Modul zugeführt wird, dem pH-Wert des Fluids, welches aus dem zweiten Modul abgeführt wird, entsprechen oder auch davon verschieden sein.

Gemäß einer besonders bevorzugten Ausführungsform der vorliegenden Erfindung wird im ersten Modul der pH-Wert des Fluids abgesenkt, wobei im zweiten Modul der pH-Wert des Fluids erhöht wird. Durch diese besondere Ausgestaltung des erfindungsgemäßen Verfahrens können im Fluid gegebenenfalls als Verunreinigungen enthaltene Viren inaktiviert werden. "Inaktiviert" bedeutet, dass die Viren nicht mehr zur Vermehrung fähig sind. Vorzugsweise wird der pH auf einen Wert von 2 bis 6, besonders bevorzugt von 3 bis 5, abgesenkt.

Wie vorstehend beschrieben, ist gemäß einer bevorzugten Ausführungsform der vorliegenden Erfindung das erste Modul ein Membranadsorber und das zweite Modul die Filtereinheit mit erstem und zweitem Filtermedium. In diesem Fall umfasst das erfindungsgemäße Verfahren vorzugsweise nach Schritt (a) und vor Schritt (b) die Schritte (a') Zuführen eines Beladungsmediums in das erste Modul und (a") Abführen des Beladungsmediums aus dem ersten Modul. Das Beladungsmedium kann die Vorrichtung über das Verweilmodul und das zweite Modul (Filtereinheit) verlassen. Vorzugsweise ist am Auslass des Membranadsorbers ein Ventil angebracht, sodass das Beladungsmedium darüber abgeführt werden kann, ohne das Verweilmodul und das zweite Modul passieren zu müssen.

Es ist besonders bevorzugt, dass das Fluid ein Gemisch ist, welches einen wässrigen Puffer sowie ein oder mehrere Produkte und gegebenenfalls eine oder mehrere Verunreinigungen umfasst bzw. daraus besteht. Gemäß einer besonders bevorzugten Ausführungsform des erfindungsgemäßen Verfahrens enthält das dem ersten Modul zugeführte Fluid einen wässrigen Puffer, einen oder mehrere monoklonale Antikörper sowie gegebenenfalls Viren und gegebenenfalls eine oder mehrere Verunreinigungen.

Erfindungsgemäß kann durch das Einstellen des Volumenstroms am Einlass und am Auslass des Retentatkanals das Volumen des Fluids und damit die Konzentration der darin enthaltenen Bestandteile eingestellt werden. Vorzugsweise beträgt das Verhältnis des Volumenstroms des in Schritt (ii) zugeführten Fluids zu dem Volumenstrom des in Schritt (iii) abgeführten Fluids 1 : 10 bis 10 : 1. Darüber hinaus beträgt das Verhältnis des Volumenstroms des in Schritt (i) zugeführten Versorgungsmediums zu dem Volumenstrom des in Schritt (ii) zugeführten Fluids vorzugsweise 1 : 1000 bis 10 : 1.

In einer bevorzugten Ausgestaltung des Verfahrens wird das Versorgungsmedium mit einem Druck von 0,1 bis 4 bar zugeführt. Besonders bevorzugt wird das Versorgungsmedium mit einem Druck zugeführt, der größer ist als der Retentatausgangsdruck. Weiterhin beträgt die Druckdifferenz zwischen Retentatkanal und Permeatkanal vorzugsweise 0,1 bis 1,5 bar.

Gemäß einer bevorzugten Ausführungsform der vorliegenden Erfindung beträgt die Verweilzeit des Fluids in dem Verweilmodul 1 Minute bis 24 Stunden, vorzugsweise 5 Minuten bis 2 Stunden, noch bevorzugter 10 Minuten bis 1 Stunde.

Gemäß einer bevorzugten Ausführungsform der vorliegenden Erfindung ist das erste Modul eine erste Filtereinheit und das zweite Modul eine zweite Filtereinheit, wobei Schritt (b) die folgenden Schritte umfasst: (b-i) Zuführen des Versorgungsmediums in den Einlass für das Versorgungsmedium der ersten Filtereinheit; (b-ii) Zuführen des Fluids in den Einlass für das Fluid der ersten Filtereinheit; (b-iii) Abführen des Fluids aus dem Auslass für das Fluid der ersten Filtereinheit; und (b-iv) Abführen des Permeats aus dem Auslass für das Permeat der ersten Filtereinheit und Schritt (c) die folgenden Schritte umfasst: (c-i) Zuführen des Versorgungsmediums in den Einlass für das Versorgungsmedium der zweiten Filtereinheit; (c-ii) Zuführen des Fluids in den Einlass für das Fluid der zweiten Filtereinheit; (c-iii) Abführen des Fluids aus dem Auslass für das Fluid der zweiten Filtereinheit; und Abführen des Permeats aus dem Auslass für das Permeat der zweiten Filtereinheit. Das in Schritt (b-iii) abgeführte Fluid wird dem Einlass des Verweilmoduls zugeführt. Das aus dem Verweilmodul abgeführte Fluid wird in Schritt (c-ii) in den Einlass für das Fluid der zweiten Filtereinheit zugeführt.

Vorzugsweise wird das erfindungsgemäße Verfahren kontinuierlich betrieben, das heißt unter konstanter/kontinuierlicher Zugabe des Versorgungsmediums und des Fluids, wodurch ein besonders effizientes und wirtschaftliches Verfahren zur mehrfachen Änderung der Konzentration eines Fluids bereitgestellt werden kann. Erfindungsgemäß wird unter einem "kontinuierlichen" Verfahren zur mehrfachen Änderung der Konzentration eines Fluids ein Verfahren verstanden, in dem sowohl das Versorgungsmedium als auch das Fluid kontinuierlich zugegeben werden.
Figuren 1 und 2 zeigen beispielhafte Ausführungsformen der erfindungsgemäßen Vorrichtung bzw. des erfindungsgemäßen Verfahrens.
Figur 3 zeigt die Ergebnisse des Referenzbeispiels.

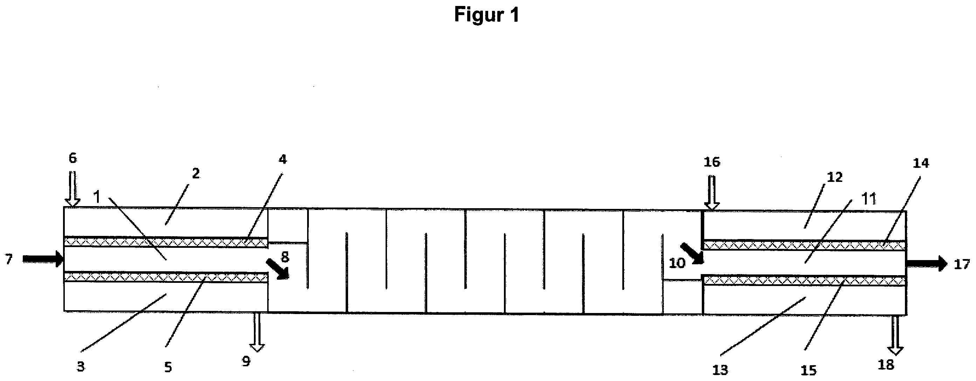

Eine beispielhafte Ausführungsform der erfindungsgemäßen Vorrichtung bzw. des erfindungsgemäßen Verfahrens ist in Figur 1 gezeigt. Die Vorrichtung dieser Ausführungsform ist beispielsweise dazu geeignet, den pH-Wert eines Fluids einzustellen. Die Vorrichtung weist drei Module auf: Ein erstes Modul (links), welches direkt in ein Verweilmodul (mittig) übergeht, welches wiederum direkt in ein zweites Modul (rechts) übergeht.

Jedes von dem ersten und zweiten Modul ist eine Filtereinheit. Jede der Filtereinheiten weist einen Retentatkanal (1, 11), einen Versorgungskanal (2, 12), einen Permeatkanal (3, 13), ein erstes Filtermedium (4, 14) und ein zweites Filtermedium (5, 15) auf. Das erste Filtermedium (4, 14) trennt den Versorgungskanal (2, 12) vom Retentatkanal (1, 11) und das zweite Filtermedium (5, 15) trennt den Retentatkanal (1, 11) vom Permeatkanal (3, 13).

Über den Einlass (7, 10) des Retentatkanals (1, 11) der Filtereinheit des ersten Moduls (erste Filtereinheit) bzw. der Filtereinheit des zweiten Moduls (zweite Filtereinheit) wird das Fluid dem ersten bzw. zweiten Modul zugeführt. Über den Einlass (6, 16) des Versorgungskanals (2, 12) wird ein Versorgungsmedium zugeführt. Das Versorgungsmedium passiert das erste Filtermedium (4, 14) und wird so mit dem Fluid im Retentatkanal (1, 11) in Kontakt gebracht. Der ersten und zweiten Filtereinheit kann erfindungsgemäß das gleiche Versorgungsmedium zugeführt werden, wobei vorzugsweise Versorgungsmedien mit unterschiedlicher Zusammensetzung verwendet werden. Über den Versorgungskanal (2, 12) wird ein Fluid zugeführt, welches die Zusammensetzung des Fluids, z. B. dessen pH-Wert, im Retentat verändert. Im Fall der Virusinaktivierung kann als erstes Versorgungsmedium über den Einlass (6) des Versorgungskanals der ersten Filtereinheit eine saure wässrige Lösung zugeführt werden, beispielsweise eine Lösung von Zitronensäure in Wasser, um eine Absenkung des pH-Wertes des Fluids im Retentatkanal (1) zu bewirken. Als zweites Versorgungsmedium kann über den Einlass (16) des Versorgungskanals der zweiten Filtereinheit eine Pufferlösung (z. B. wässriger Phosphatpuffer) oder eine basische wässrige Lösung zugeführt werden, wodurch sich der pH-Wert des Fluids im Retentatkanal (11) der zweiten Filtereinheit erhöht.

Die Eigenschaften des zweiten Filtermediums (5, 15) können derart gewählt werden, dass nur bestimmte Bestandteile des Fluids das zweite Filtermedium (5, 15) passieren, um so einen gewünschten Separationseffekt zu erzielen. Insbesondere wenn das Fluid als Produkt einen oder mehrere Antikörper enthält, kann eine Ultrafiltrationsmembran mit einer Porengröße von 2 bis 100 nm als zweites Filtermedium (5, 15) verwendet werden, damit der oder die Antikörper im Fluid verbleiben und nicht in den Permeatkanal (3, 13) gelangen. Darüber hinaus kann durch Verwendung einer Ultrafiltrationsmembran mit einer Porengröße von 2 bis 100 nm als zweites Filtermedium (5, 15) ein im Fluid als Produkt enthaltenes Vakzin durch die Ultrafiltrationsmembran zurückgehalten werden. Bestandteile des Fluids, welche das zweite Filtermedium (5, 15) passieren können (z. B. Verunreinigungen), passieren zumindest teilweise oder vollständig das zweite Filtermedium (5, 15) und werden über den Permeatkanal (3, 13) und dessen Auslass (9, 18) als Permeat abgeführt. Dadurch können neben einer mehrmaligen Änderung der Zusammensetzung des Fluids Verunreinigungen abgetrennt werden.

Über den Auslass (8) des Retentatkanals (1) der ersten Filtereinheit bzw. den Einlass (8) des Verweilmoduls wird das Fluid vom ersten Modul dem Verweilmodul zugeführt. Das Verweilmodul weist eine Mehrzahl von Umlenkplatten auf, welche einen Fluidpfad definieren, der von dem Fluid durchströmt wird. Über die Gestaltung des Fluidpfads (Pfadlänge, Flussgeschwindigkeit des Fluids und Anströmfläche des Pfads) kann die Verweilzeit des Fluids im Verweilmodul eingestellt werden. So bleiben beispielsweise im Fall der Virusinaktivierung während dieser Verweilzeit die Viren den im ersten Modul eingestellten Eigenschaften des Fluids (beispielsweise niedriger pH-Wert) ausgesetzt, wodurch eine vollständige Inaktivierung der Viren gewährleistet werden kann. Das Fluid verlässt das Verweilmodul über dessen Auslass (10) bzw. den Einlass (10) des Retentatkanals der zweiten Filtereinheit.

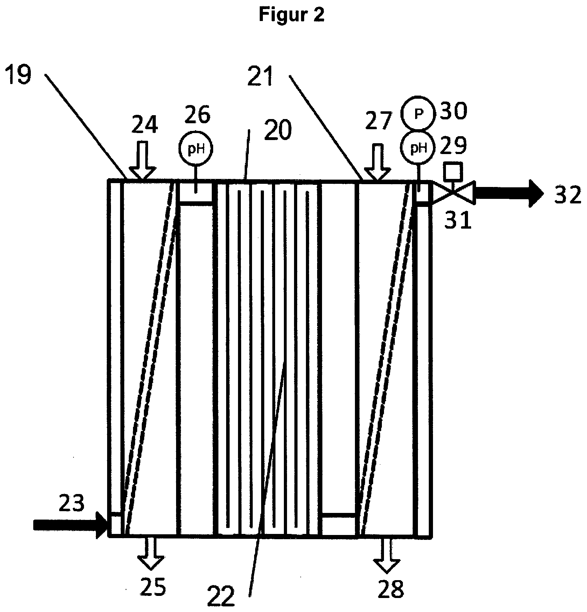

Eine weitere beispielhafte Ausführungsform der erfindungsgemäßen Vorrichtung bzw. des erfindungsgemäßen Verfahrens ist in Figur 2 gezeigt. Auch die in Figur 2 dargestellte Ausführungsform ist für die Virusinaktivierung und insbesondere zur Virusinaktivierung eines Fluids, welches monoklonale Antikörper enthält, unter gleichzeitiger Entfernung von Verunreinigungen aus dem Fluid (über das Permeat) geeignet.

Das Fluid wird in dieser Ausführungsform über einen Druckgradienten in den Retentatkanal eingebracht, zeitgleich findet über einen Druckgradienten zwischen Retentatkanal und Permeatkanal eine Filtration in den Permeatkanal statt. Der Druckgradient kann beispielsweise über ein Ventil am Auslass des Retentatkanals des ersten Moduls (19) bzw. der ersten Filtereinheit (19) und/oder über ein Ventil am Auslass des Retentatkanals des zweiten Moduls (21) bzw. der zweiten Filtereinheit (21) und/oder über ein Ventil am Auslass des Verweilmoduls (20) gesteuert werden.

Das Fluid kann aus einem gegebenenfalls vorgelagerten Chromatographieschritt mithilfe einer Pumpe (in Figur 2 nicht dargestellt) in den Einlass (23) des Retentatkanals der ersten Filtereinheit (19) eingebracht werden. Über die Auswahl einer geeigneten Trenngrenze des zweiten Filtermediums der Filtereinheiten (19, 21) kann ein im Fluid enthaltenes Produkt/Zielmolekül (z. B. monoklonaler Antikörper) zurückgehalten werden, ohne das zweite Filtermedium zu passieren.

Über den Einlass (24) des Versorgungskanals wird ein Versorgungsmedium (beispielsweise eine wässrige Zitronensäurelösung) in den Versorgungskanal und damit über das erste Filtermedium in den Retentatkanal eingebracht. Die zugeführte Menge wird über den mittels eines pH-Sensors (26) gemessenen pH-Wert gesteuert. Der pH-Wert kann beispielsweise von 7 auf 3,5 gesenkt werden.

Simultan findet in dieser bevorzugten Ausführungsform aufgrund eines Druckgradienten zwischen Retentatkanal und Permeatkanal eine Filtration in den Permeatkanal statt. Der Volumenstrom am Auslass (25) des Permeatkanals ist im Ausführungsbeispiel gleich groß wie der Volumenstrom der dem Versorgungskanal zugeführten Menge an Versorgungsmedium. Damit bleiben die Konzentration der im Fluid verbleibenden Bestandteile sowie das Volumen des Fluids konstant.

Bevorzugt wird als zweites Filtermedium ein Ultrafilter mit einer Porengröße im Bereich von 2 bis 100 nm eingesetzt. Erstes und zweites Filtermedium müssen nicht die gleiche Porengröße und/oder Materialeigenschaften aufweisen. Vorzugsweise beträgt der Transmembrandruck zwischen Retentatkanal und Permeatkanal 0,1 bis 1,5 bar. Der Transmembrandruck zwischen Versorgungskanal und Retentatkanal beträgt vorzugsweise 0,01 bis 0,4 bar.

Das Verweilmodul ist in dem in Figur 2 dargestellten Ausführungsbeispiel direkt mit dem erstem Modul / der ersten Filtereinheit verbunden. Im Verweilmodul dieses Ausführungsbeispiels wird durch Anordnen von Umlenkfolien (22) und Abstandshaltern (z. B. Geweben; in Figur 2 nicht dargestellt) zu Stapeln ein Kanal gebildet. Dieser Kanal bildet einen Fluidpfad. In Übereinstimmung mit üblichen Flachfilterbauweisen, die dem Fachmann bekannt sind, können die Stapel beispielsweise durch Verpressen, Umspritzen oder Verguss kombiniert werden.

Im zweiten Modul der in Figur 2 dargestellten Ausführungsform wird analog zum ersten Modul über den Einlass (27) des Versorgungskanals beispielsweise Phosphat-gepufferte Salzlösung mit einem pH-Wert von 7 in den Versorgungskanal und damit über das erste Filtermedium in den Retentatkanal eingebracht. Die zugeführte Menge kann auf Grundlage des pH-Werts eingestellt werden. Der pH-Wert kann mit einem Sensor (29) gemessenen werden. Der pH-Wert kann beispielsweise von 3,5 auf einen Wert von 7 erhöht werden.

Mit Hilfe eines Drucksensors (30) und eines Ventils (31) können der Filtrationsdruck sowie der Ausgangsvolumenstrom eingestellt werden.

In dem in Figur 2 dargestellten Ausführungsbeispiel kann im Falle eines (Perfusi-ons)prozesses mit einem Ansatzvolumen von 50 L die Fläche der Filtermedien des ersten und zweiten Moduls jeweils 1,5 m² und die Gesamtkanallänge (Fluidpfadlänge) des Verweilmoduls 2 m betragen, sofern von einem kontinuierlichen Volumenstrom von ca. 35 mL/min für eine Gesamtdauer des (Perfusions)prozesses von 24h ausgegangen wird. Die Verweilzeit des Fluids im Verweilmodul bei einem pH-Wert von 3,5 würde in diesem Fall ca. 42 min betragen.

### Bezugszeichenliste

- 1, 11: Retentatkanal
- 2, 12: Versorgungskanal
- 3, 13: Permeatkanal
- 4, 14: erstes Filtermedium
- 5, 15: zweites Filtermedium
- 6, 16, 24, 27: Einlass für Versorgungsmedium
- 7, 10, 23: Einlass des Retentatkanals für das Fluid
- 8: Einlass des Verweilmoduls
- 8, 17, 32: Auslass für das Fluid
- 9, 18, 25, 28: Auslass für das Permeat
- 10: Auslass des Verweilmoduls
- 19: erstes Modul
- 20: Verweilmodul
- 21: zweites Modul
- 22: Umlenkfolien
- 26, 29: pH-Sensor
- 30: Drucksensor
- 31: Ventil

Die vorliegende Erfindung wird weiter durch das nachfolgende Referenzbeispiel erläutert, ohne darauf beschränkt zu sein.

### Referenzbeispiel

Es wurden folgende Ausgansmaterialien bereitgestellt.

| | |
|---|---|
| Speiseflüssigkeit (Fluid): | 20 g/L Rinderserumalbumin (BSA) in 0,1 M Citratpuffer mit einem pH-Wert von 3,5 |
| Versorgungsflüssigkeit: | 0,1 M Citratpuffer mit einem pH-Wert von 2 |

Ziel: Das Umpuffern (Pufferaustausch) der Speiseflüssigkeit und eine pH-Wert-Absenkung von pH 3,5 auf pH 2,5 mit Hilfe der Versorgungsflüssigkeit und eine Inkubation in einem Verweilmodul.

Als erstes Modul wurde eine erste Filtereinheit mit erstem und zweitem Filtermedium vom Typ Ultrafiltermembran aus Polyethersulfon mit einem MWCO (molecular weight cut-off, Molekülgewichtsausschlussgrenze) von 10 kDa verwendet. Die Gesamtmembranfläche des ersten Filtermediums betrug 0,027 m². In den Retentatkanal der ersten Filtereinheit war ein Gewebe zur Durchmischung des Retentats eingebracht.

Zur Überwachung des Prozesses wurde der Transmembrandruck (TMP) gemessen. Weiterhin wurde die Proteinkonzentration des Retentats am Retentatauslass gemessen, um festzustellen, wann die Konzentration von BSA im Retentat derjenigen in der Speiseflüssigkeit entsprach und der Prozess im Gleichgewichtszustand war. Weiterhin wurde der pH-Wert des Retentats am Retentatauslass gemessen, um festzustellen, wann der pH-Wert des Retentats der Zielvorgabe von pH 2,5 entsprach und der Prozess im Gleichgewichtszustand war. Der jeweilige Volumenstrom der Speiseflüssigkeit, der Versorgungsflüssigkeit und des Retentats betrug 6 mL/min, 6,5 mL/min bzw. 6 mL/min.

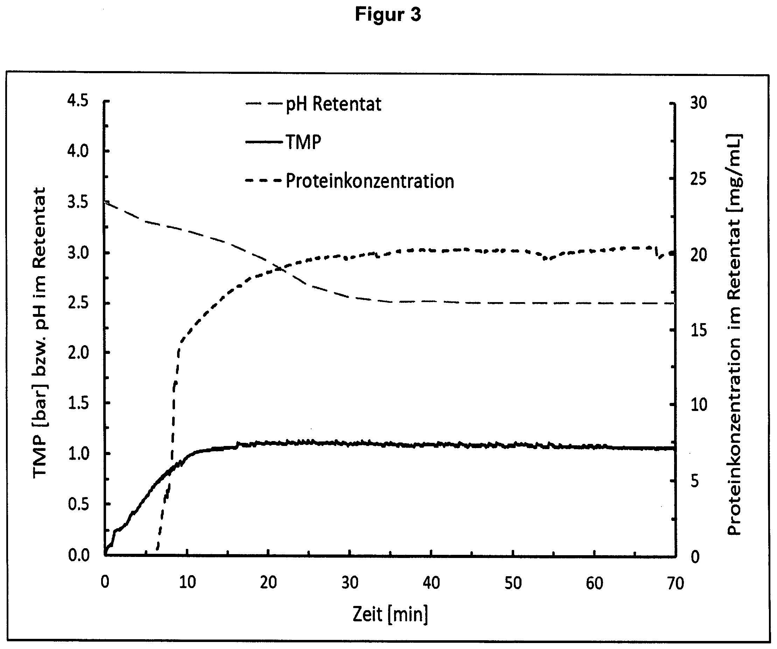

Figur 3 zeigt, dass ein Gleichgewichtszustand für alle Parameter TMP, pH und Proteinkonzentration erreicht werden konnte. Der geforderte pH-Wert von 2,5 im Retentat wurde erreicht. Bedingt durch das Gewebe im Retentatkanal wird das Retentat optimal durchmischt.

Das Retentat wurde anschließend in ein Verweilmodul geleitet, das aus 50 aufeinanderfolgenden Kanälen, wie schematisch in Figur 2 dargestellt, bestand. Die Kanäle wurden jeweils durch eine Umlenkfolie unterteilt. Die Höhe, die Breite und die Länge eines jeden Kanals betrug etwa 0,41 mm, 30 mm bzw. 150 mm. Damit ergab sich eine Gesamtlänge des vom Verweilmodul definierten Fluidpfads von etwa 7500 mm. Die Verweilzeit des Fluids im Verweilmodul wurde bestimmt, indem die Zeit vom Eintritt der Flüssigkeit in den Eingang bis zum Austritt der Flüssigkeit aus dem Ausgang gemessen wurde. Bei einem Eingangsvolumenstrom in das Verweilmodul von 6 mL/min wurde eine Verweilzeit von 15,5 min gemessen.

## Patentansprüche

1. Vorrichtung zur mehrfachen Änderung der Zusammensetzung eines Fluids, umfassend
ein erstes Modul (19) zur Änderung der Zusammensetzung des Fluids, ein zweites Modul (21) zur Änderung der Zusammensetzung des Fluids und ein Verweilmodul (20) mit einem Einlass (8) und einem Auslass (10), wobei das Verweilmodul (20) einen Fluidpfad definiert, der
(I) einen oder mehrere mäanderförmige Kanäle und/oder einen oder mehrere spiralförmige Kanäle aufweist,
(II) durch mindestens ein Unterteilungselement unterteilt ist, und/oder
(III) mehrere parallele Strömungskanäle, die mit einem gemeinsamen Einlass und mit einem gemeinsamen Auslass für das Fluid verbunden sind, aufweist, wobei das Verweilmodul (20) so eingerichtet ist, dass die Anzahl der parallelen Strömungskanäle, durch die das Fluid fließt, vorzugsweise mithilfe von Ventilen, geändert werden kann,
und das Verweilmodul (20) so eingerichtet ist, dass die Verweilzeit des Fluids in dem Verweilmodul (20) 1 Minute bis 24 Stunden beträgt,
wobei das erste Modul (19) mit dem Einlass (8) des Verweilmoduls (20) fluidleitend verbunden ist und der Auslass (10) des Verweilmoduls (20) fluidleitend mit dem zweiten Modul (21) verbunden ist,
wobei das erste Modul (19) oder das zweite Modul (21) eine Filtereinheit ist oder das erste Modul (19) eine erste Filtereinheit ist und das zweite Modul (21) eine zweite Filtereinheit ist, die Filtereinheit beziehungsweise Filtereinheiten jeweils aufweisend
mindestens einen Versorgungskanal (2, 12),
mindestens ein erstes Filtermedium (4, 14),
mindestens einen Retentatkanal (1, 11),
mindestens ein zweites Filtermedium (5, 15) und
mindestens einen Permeatkanal (3, 13),
derart angeordnet, dass das erste Filtermedium (4, 14) den Versorgungskanal (2, 12) und den Retentatkanal (1, 11) voneinander abgrenzt und das zweite Filtermedium (5, 15) den Retentatkanal (1, 11) und den Permeatkanal (3, 13) voneinander abgrenzt, wobei
der Versorgungskanal (2, 12) fluidleitend mit mindestens einem Einlass (6, 16, 24, 27) für ein Versorgungsmedium verbunden ist,
der Retentatkanal (1, 11) fluidleitend mit mindestens einem Einlass (7, 10, 23) für das Fluid und mit mindestens einem Auslass (8, 17, 32) für das Fluid verbunden ist, und
der Permeatkanal (3, 13) fluidleitend mit mindestens einem Auslass (9, 18, 25, 28) für ein Permeat verbunden ist.

2. Vorrichtung nach Anspruch 1,
wobei das erste Modul (19) eine erste Filtereinheit ist und das zweite Modul (21) eine zweite Filtereinheit ist.

3. Vorrichtung nach Anspruch 1,
wobei das erste oder zweite Modul (19, 21) ein statischer Mischer ist.

4. Verfahren zur mehrfachen Änderung der Zusammensetzung eines Fluids, umfassend
(a) Bereitstellen der Vorrichtung nach einem der Ansprüche 1 bis 3;
(b) Zuführen des Fluids in das erste Modul (19); und
(c) Abführen des Fluids aus dem zweiten Modul (21),
wobei Schritt (b) und/oder Schritt (c) die folgenden Schritte umfasst/umfassen:
(i) Zuführen des Versorgungsmediums in den Einlass (6, 16, 24, 27) für das Versorgungsmedium;
(ii) Zuführen des Fluids in den Einlass (7, 10, 23) für das Fluid;
(iii) Abführen des Fluids aus dem Auslass (8, 17, 32) für das Fluid; und
(iv) Abführen des Permeats aus dem Auslass (9, 18, 25, 28) für das Permeat,
wobei das Verweilmodul (20) einen Fluidpfad definiert, der
(I) einen oder mehrere mäanderförmige Kanäle und/oder einen oder mehrere spiralförmige Kanäle aufweist,
(II) durch mindestens ein Unterteilungselement unterteilt ist, und/oder
(III) mehrere parallele Strömungskanäle, die mit einem gemeinsamen Einlass und mit einem gemeinsamen Auslass für das Fluid verbunden sind, aufweist, wobei das Verweilmodul (20) so eingerichtet ist, dass die Anzahl der parallelen Strömungskanäle, durch die das Fluid fließt, vorzugsweise mithilfe von Ventilen, geändert werden kann,
und die Verweilzeit des Fluids in dem Verweilmodul (20) 1 Minute bis 24 Stunden beträgt.

5. Verfahren nach Anspruch 4,
wobei das Fluid, welches in das erste Modul (19) eingeleitet wird, ein oder mehrere Produkte sowie gegebenenfalls eine oder mehrere Verunreinigungen enthält.

6. Verfahren nach Anspruch 4 oder 5,
wobei im ersten Modul (19) der pH-Wert des Fluids abgesenkt bzw. erhöht wird und im zweiten Modul (21) der pH-Wert des Fluids erhöht bzw. abgesenkt wird.

7. Verfahren nach Anspruch 6,
wobei im ersten Modul (19) der pH-Wert des Fluids abgesenkt wird und im zweiten Modul (21) der pH-Wert des Fluids erhöht wird.

8. Verfahren nach einem der Ansprüche 4 bis 7,
wobei das erste Modul (19) eine erste Filtereinheit ist und das zweite Modul (21) eine zweite Filtereinheit ist,
Schritt (b) die folgenden Schritte umfasst:
(b-i) Zuführen des Versorgungsmediums in den Einlass (6, 24) für das Versorgungsmedium der ersten Filtereinheit;
(b-ii) Zuführen des Fluids in den Einlass (7, 23) für das Fluid der ersten Filtereinheit;
(b-iii) Abführen des Fluids aus dem Auslass (8) für das Fluid der ersten Filtereinheit; und
(b-iv) Abführen des Permeats aus dem Auslass (9, 25) für das Permeat der ersten Filtereinheit und
Schritt (c) die folgenden Schritte umfasst:
(c-i) Zuführen des Versorgungsmediums in den Einlass (16, 27) für das Versorgungsmedium der zweiten Filtereinheit;
(c-ii) Zuführen des Fluids in den Einlass (10) für das Fluid der zweiten Filtereinheit;
(c-iii) Abführen des Fluids aus dem Auslass (17, 32) für das Fluid der zweiten Filtereinheit; und
(c-iv) Abführen des Permeats aus dem Auslass (18, 28) für das Permeat der zweiten Filtereinheit.

9. Vorrichtung nach Anspruch 1 oder Verfahren nach Anspruch 4, wobei bei (I) und (III) ein Abstandshalter, vorzugsweise ein Textilmaterial, in den von dem Verweilmodul (20) definierten Fluidpfad eingebracht ist.

10. Vorrichtung nach Anspruch 1 oder Verfahren nach Anspruch 4, wobei das mindestens eine Unterteilungselement aus (II) aus einer Mehrzahl von Umlenkplatten und/oder Umlenkfolien besteht.

11. Vorrichtung nach Anspruch 10 oder Verfahren nach Anspruch 10, wobei zwischen den Umlenkfolien Abstandshalter, vorzugsweise ein Textilmaterial, eingebracht sind.

12. Verwendung der Vorrichtung nach einem der Ansprüche 1 bis 3 zur
(A) Virusinaktivierung,
(B) Durchführung einer katalytischen Reaktion, wobei ein Katalysator im ersten Modul (19) zum Fluid gegeben wird, im Verweilmodul (20) eine durch den Katalysator katalysierte Reaktion abläuft und der Katalysator im zweiten Modul (21) vom Fluid abgetrennt wird, oder
(C) Änderung des pH-Wertes, wobei im ersten Modul (19) der pH-Wert des Fluids abgesenkt bzw. erhöht und im zweiten Modul (21) der pH-Wert des Fluids erhöht bzw. abgesenkt wird.

13. Verwendung der Vorrichtung nach einem der Ansprüche 1 bis 3 zur Durchführung einer Fällungsreaktion, welche im ersten Modul (19) ausgelöst und dessen Fällungsprodukt im zweiten Modul (21) abgetrennt wird, wobei das erste (19) oder zweite Modul (21) die Filtereinheit mit erstem und zweitem Filtermedium ist und das zweite (21) oder erste Modul (19) eine weitere Filtereinheit, aufweisend mindestens ein Filtermedium, ist.

## Claims

1. Device for the multiple modification of the composition of a fluid, comprising a first module (19) for modifying the composition of the fluid, a second module (21) for modifying the composition of the fluid and a residence module (20) with an inlet (8) and an outlet (10), the residence module (20) defining a fluid path which
(I) has one or more meandering channels and/or one or more spiral channels,
(II) is subdivided by at least one subdivision element, and/or
(III) has several parallel flow channels which are connected to a common inlet and a common outlet for the fluid, the residence module (20) being arranged in such a way that the number of parallel flow channels through which the fluid flows can be modified, preferably by means of valves,
and the residence module (20) being arranged in such a way that the residence time of the fluid in the residence module (20) is 1 minute to 24 hours,
the first module (19) being fluidically connected to the inlet (8) of the residence module (20) and the outlet (10) of the residence module (20) being fluidically connected to the second module (21),
the first module (19) or the second module (21) being a filter unit or the first module (19) being a first filter unit and the second module (21) being a second filter unit, the filter unit or filter units each having
at least one supply channel (2, 12),
at least one first filter medium (4, 14),
at least one retentate channel (1, 11),
at least one second filter medium (5, 15) and
at least one permeate channel (3, 13),
arranged in such a way that the first filter medium (4, 14) separates the supply channel (2, 12) and the retentate channel (1, 11) from one another and the second filter medium (5, 15) separates the retentate channel (1, 11) and the permeate channel (3, 13) from one another, wherein
the supply channel (2, 12) is fluidically connected to at least one inlet (6, 16, 24, 27) for a supply medium,
the retentate channel (1, 11) is fluidically connected to at least one inlet (7, 10, 23) for the fluid and to at least one outlet (8, 17, 32) for the fluid, and
the permeate channel (3, 13) is fluidically connected to at least one outlet (9, 18, 25, 28) for a permeate.

2. Device according to claim 1,
wherein the first module (19) is a first filter unit and the second module (21) is a second filter unit.

3. Device according to claim 1,
wherein the first or second module (19, 21) is a static mixer.

4. Method for the multiple modification of the composition of a fluid, comprising
(a) providing the device according to any one of claims 1 to 3;
(b) feeding the fluid into the first module (19); and
(c) discharging the fluid out of the second module (21),
wherein step (b) and/or step (c) comprises/comprise the following steps:
(i) feeding the supply medium into the inlet (6, 16, 24, 27) for the supply medium;
(ii) feeding the fluid into the inlet (7, 10, 23) for the fluid;
(iii) discharging the fluid out of the outlet (8, 17, 32) for the fluid; and
(iv) discharging the permeate out of the outlet (9, 18, 25, 28) for the permeate,
wherein the residence module (20) defines a fluid path which
(I) has one or more meandering channels and/or one or more spiral channels,
(II) is subdivided by at least one subdivision element, and/or
(III) has several parallel flow channels which are connected to a common inlet and a common outlet for the fluid, the residence module (20) being arranged in such a way that the number of parallel flow channels through which the fluid flows can be modified, preferably by means of valves,
and the residence time of the fluid in the residence module (20) is 1 minute to 24 hours.

5. Method according to claim 4,
wherein the fluid which is introduced into the first module (19) contains one or more products as well as optionally one or more impurities.

6. Method according to claim 4 or 5,
wherein, in the first module (19), the pH value of the fluid is lowered or raised and, in the second module (21), the pH value of the fluid is raised or lowered.

7. Method according to claim 6,
wherein, in the first module (19), the pH value of the fluid is lowered and, in the second module (21), the pH value of the fluid is raised.

8. Method according to any one of claims 4 to 7,
wherein the first module (19) is a first filter unit and the second module (21) is a second filter unit,
step (b) comprises the following steps:
(b-i) feeding the supply medium into the inlet (6, 24) for the supply medium of the first filter unit;
(b-ii) feeding the fluid into the inlet (7, 23) for the fluid of the first filter unit;
(b-iii) discharging the fluid out of the outlet (8) for the fluid of the first filter unit; and
(b-iv) discharging the permeate out of the outlet (9, 25) for the permeate of the first filter unit and
step (c) comprises the following steps:
(c-i) feeding the supply medium into the inlet (16, 27) for the supply medium of the second filter unit;
(c-ii) feeding the fluid into the inlet (10) for the fluid of the second filter unit;
(c-iii) discharging the fluid out of the outlet (17, 32) for the fluid of the second filter unit; and
(c-iv) discharging the permeate out of the outlet (18, 28) for the permeate of the second filter unit.

9. Device according to claim 1 or method according to claim 4, wherein, at (I) and (III), a spacer, preferably a textile material, is introduced into the fluid path defined by the residence module (20).

10. Device according to claim 1 or method according to claim 4, wherein the at least one subdivision element of (II) consists of a plurality of baffle plates and/or baffle foils.

11. Device according to claim 10 or method according to claim 10, wherein spacers, preferably a textile material, are introduced between the baffle foils.

12. Use of the device according to any one of claims 1 to 3 for
(A) virus inactivation,
(B) carrying out a catalytic reaction, wherein a catalyst is added to the fluid in the first module (19), a reaction catalysed by the catalyst takes place in the residence module (20) and the catalyst is separated from the fluid in the second module (21), or
(C) modification of the pH value, wherein, in the first module (19), the pH value of the fluid is lowered or raised and, in the second module (21), the pH value of the fluid is raised or lowered.

13. Use of the device according to any one of claims 1 to 3 for carrying out a precipitation reaction which is triggered in the first module (19) and the precipitation product of which is separated in the second module (21), wherein the first (19) or second module (21) is the filter unit with first and second filter medium and the second (21) or first module (19) is a further filter unit, having at least one filter medium.

## Revendications

1. Dispositif pour la modification multiple de la composition d'un fluide, comprenant un premier module (19) pour modifier la composition du fluide, un deuxième module (21) pour modifier la composition du fluide et un module de séjour (20) avec une entrée (8) et une sortie (10), le module de séjour (20) définissant un trajet de fluide qui
(I) présente un ou plusieurs canaux méandriques et/ou un ou plusieurs canaux spiralés,
(II) est subdivisé par au moins un élément de subdivision, et/ou
(III) présente plusieurs canaux d'écoulement parallèles qui sont reliés à une entrée commune et à une sortie commune pour le fluide, le module de séjour (20) étant agencé de telle sorte que le nombre des canaux d'écoulement parallèles à travers lesquels s'écoule le fluide peut être modifié, de préférence à l'aide de vannes,
et le module de séjour (20) étant agencé de telle sorte que le temps de séjour du fluide dans le module de séjour (20) est de 1 minute à 24 heures,
le premier module (19) étant relié de manière conductrice de fluide à l'entrée (8) du module de séjour (20) et la sortie (10) du module de séjour (20) étant reliée de manière conductrice de fluide au deuxième module (21),
le premier module (19) ou le deuxième module (21) étant une unité de filtration ou le premier module (19) étant une première unité de filtration et le deuxième module (21) étant une deuxième unité de filtration, l'unité de filtration ou les unités de filtration présentant chacune
au moins un canal d'alimentation (2, 12),
au moins un premier milieu filtrant (4, 14),
au moins un canal de rétentat (1, 11),
au moins un deuxième milieu filtrant (5, 15) et
au moins un canal de perméat (3, 13),
agencés de telle sorte que le premier milieu filtrant (4, 14) sépare le canal d'alimentation (2, 12) et le canal de rétentat (1, 11) l'un de l'autre et le deuxième milieu filtrant (5, 15) sépare le canal de rétentat (1, 11) et le canal de perméat (3, 13) l'un de l'autre,
le canal d'alimentation (2, 12) étant relié de manière conductrice de fluide à au moins une entrée (6, 16, 24, 27) pour un milieu d'alimentation,
le canal de rétentat (1, 11) étant relié de manière conductrice de fluide à au moins une entrée (7, 10, 23) pour le fluide et à au moins une sortie (8, 17, 32) pour le fluide, et
le canal de perméat (3, 13) étant relié de manière conductrice de fluide à au moins une sortie (9, 18, 25, 28) pour un perméat.

2. Dispositif selon la revendication 1,
dans lequel le premier module (19) est une première unité de filtration et le deuxième module (21) est une deuxième unité de filtration.

3. Dispositif selon la revendication 1,
dans lequel le premier ou deuxième module (19, 21) est un mélangeur statique.

4. Procédé pour la modification multiple de la composition d'un fluide, comprenant
(a) la fourniture du dispositif selon l'une des revendications 1 à 3 ;
(b) l'amenée du fluide dans le premier module (19) ; et
(c) l'évacuation du fluide hors du deuxième module (21),
l'étape (b) et/ou l'étape (c) comprenant/comprenant les étapes suivantes :
(i) amenée du milieu d'alimentation dans l'entrée (6, 16, 24, 27) pour le milieu d'alimentation ;
(ii) amenée du fluide dans l'entrée (7, 10, 23) pour le fluide ;
(iii) évacuation du fluide hors de la sortie (8, 17, 32) pour le fluide ; et
(iv) évacuation du perméat hors de la sortie (9, 18, 25, 28) pour le perméat,
le module de séjour (20) définissant un trajet de fluide qui
(I) présente un ou plusieurs canaux méandriques et/ou un ou plusieurs canaux spiralés,
(II) est subdivisé par au moins un élément de subdivision, et/ou
(III) présente plusieurs canaux d'écoulement parallèles qui sont reliés à une entrée commune et à une sortie commune pour le fluide, le module de séjour (20) étant agencé de telle sorte que le nombre des canaux d'écoulement parallèles à travers lesquels s'écoule le fluide peut être modifié, de préférence à l'aide de vannes,
et le temps de séjour du fluide dans le module de séjour (20) est de 1 minute à 24 heures.

5. Procédé selon la revendication 4,
dans lequel le fluide qui est introduit dans le premier module (19) contient un ou plusieurs produits ainsi qu'éventuellement une ou plusieurs impuretés.

6. Procédé selon la revendication 4 ou 5,
dans lequel, dans le premier module (19), la valeur de pH du fluide est abaissée ou augmentée et, dans le deuxième module (21), la valeur de pH du fluide est augmentée ou abaissée.

7. Procédé selon la revendication 6,
dans lequel, dans le premier module (19), la valeur de pH du fluide est abaissée et, dans le deuxième module (21), la valeur de pH du fluide est augmentée.

8. Procédé selon l'une des revendications 4 à 7,
dans lequel le premier module (19) est une première unité de filtration et le deuxième module (21) est une deuxième unité de filtration,
l'étape (b) comprend les étapes suivantes :
(b-i) amenée du milieu d'alimentation dans l'entrée (6, 24) pour le milieu d'alimentation de la première unité de filtration ;
(b-ii) amenée du fluide dans l'entrée (7, 23) pour le fluide de la première unité de filtration ;
(b-iii) évacuation du fluide hors de la sortie (8) pour le fluide de la première unité de filtration ; et
(b-iv) évacuation du perméat hors de la sortie (9, 25) pour le perméat de la première unité de filtration et
l'étape (c) comprend les étapes suivantes :
(c-i) amenée du milieu d'alimentation dans l'entrée (16, 27) pour le milieu d'alimentation de la deuxième unité de filtration ;
(c-ii) amenée du fluide dans l'entrée (10) pour le fluide de la deuxième unité de filtration ;
(c-iii) évacuation du fluide hors de la sortie (17, 32) pour le fluide de la deuxième unité de filtration ; et
(c-iv) évacuation du perméat hors de la sortie (18, 28) pour le perméat de la deuxième unité de filtration.

9. Dispositif selon la revendication 1 ou procédé selon la revendication 4, dans lequel, en (I) et (III), un écarteur, de préférence un matériau textile, est introduit dans le trajet de fluide défini par le module de séjour (20).

10. Dispositif selon la revendication 1 ou procédé selon la revendication 4, dans lequel le au moins un élément de subdivision de (II) est constitué d'une pluralité de plaques de déviation et/ou de feuilles de déviation.

11. Dispositif selon la revendication 10 ou procédé selon la revendication 10, dans lequel des écarteurs, de préférence un matériau textile, sont introduits entre les feuilles de déviation.

12. Utilisation du dispositif selon l'une des revendications 1 à 3 pour
(A) l'inactivation virale,
(B) la réalisation d'une réaction catalytique, un catalyseur étant ajouté au fluide dans le premier module (19), une réaction catalysée par le catalyseur se déroulant dans le module de séjour (20) et le catalyseur étant séparé du fluide dans le deuxième module (21), ou
(C) la modification de la valeur de pH, dans laquelle, dans le premier module (19), la valeur de pH du fluide est abaissée ou augmentée et, dans le deuxième module (21), la valeur de pH du fluide est augmentée ou abaissée.

13. Utilisation du dispositif selon l'une des revendications 1 à 3 pour la réalisation d'une réaction de précipitation qui est déclenchée dans le premier module (19) et dont le produit de précipitation est séparé dans le deuxième module (21), le premier (19) ou deuxième module (21) étant l'unité de filtration avec premier et deuxième milieu filtrant et le deuxième (21) ou premier module (19) étant une autre unité de filtration, présentant au moins un milieu filtrant.
